# EUROPÄISCHE PATENTSCHRIFT

(11) **EP 0 656 021 B1**
(45) Veröffentlichungstag und Bekanntmachung des Hinweises auf die Patenterteilung: **01.10.1997**
(21) Anmeldenummer: 93915942.2
(22) Anmeldetag: 17.07.1993
(51) Int. Cl.: C08G 18/08, C08G 18/42, A61K 7/06, A61K 9/20, A61K 9/32

(54) **VERWENDUNG VON WASSERLÖSLICHEN ODER IN WASSER DISPERGIERBAREN POLYURETHANEN ALS HILFSMITTEL IN KOSMETISCHEN UND PHARMAZEUTISCHEN ZUBEREITUNGEN UND POLYURETHANE, DIE POLYMILCHSÄUREPOLYOLE EINPOLYMERISIERT ENTHALTEN**
USE OF WATER-SOLUBLE OR WATER-DISPERSIBLE POLYURETHANES AS AUXILIARY AGENTS IN COSMETIC AND PHARMACEUTICAL PREPARATIONS AND POLYURETHANES CONTAINING POLYLACTIC ACID POLYOLS INCORPORATED BY POLYMERIZATION
UTILISATION DE POLYURETHANNES SOLUBLES DANS L'EAU OU DISPERSIBLES DANS L'EAU COMME ADJUVANTS DANS DES PREPARATIONS COSMETIQUES ET PHARMACEUTIQUES, ET POLYURETHANNES RENFERMANT DES POLYOLPOLYLACTIQUES INCORPORES PAR POLYMERISATION

(30) Priorität: 29.07.1992 DE 4225045
(43) Veröffentlichungstag der Anmeldung: 07.06.1995
(60) Teilanmeldung: 97103009.3
(73) Patentinhaber: BASF Aktiengesellschaft, 67063 Ludwigshafen (DE)
(72) Erfinder: NGUYEN KIM, Son, D-6944 Hemsbach (DE); SANNER, Axel, D-6710 Frankenthal (DE); SPERLING-VIETMEIER, Karin, D-6730 Neustadt (DE)
(86) Internationale Anmeldenummer: EP9301888
(87) Internationale Veröffentlichungsnummer: WO9403510

(56) Entgegenhaltungen:
- EP-A- 0 039 162
- EP-A- 0 043 974
- DE-A- 3 814 536
- US-A- 3 835 081
- US-A- 3 975 350
- US-A- 4 743 673

## Beschreibung

Die Erfindung betrifft die Verwendung von wasserlöslichen oder in Wasser dispergierbaren Polyurethanen aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat
mit einer Glastemperatur von mindestens 15°C und Säurezahlen von 12 bis 150 oder den Salzen dieser Polyurethane in kosmetischen Zubereitungen und als Bindemittel oder Überzugsmittel in pharmazeutischen Zubereitungen.

Polyurethane, die zumindest teilweise biologisch abbaubar sind und Hydroxycarbonsäureeinheiten einpolymerisiert enthalten, sind bereits bekannt. Sie sind entweder wasserunlöslich, wie das Polyurethan aus Polymilchsäurediol und Diisocyanat, das aus der SU-A-1 016 314 bekannt ist, oder sie bilden zu weiche Filme, wie die aus der US-A-4 098 743 und der US-A-4 147 679 bekannten Polyurethane aus Poly(ε-caprolacton-diol)dimethylolpropionsäure und Diisocyanaten.

Wasserlösliche Polyurethane, die Carboxylgruppen aufweisende Diole einpolymerisiert enthalten, sind aus der US-A-3 412 054 und der US-A-3 658 939 bekannt. Sie werden als Klebstoff, Beschichtungsmittel und in Drucktinten verwendet. Sulfonat- und/oder Carboxylatgruppen enthaltende Polyurethane, die in Wasser dispergierbar sind, sind aus der DE-A-15 70 615 bekannt. Sie werden beispielsweise zur Beschichtung und zum Imprägnieren von Textilien, Leder, Papier, Holz und Metallen verwendet.

Die Patentschrift US 4,743,673 offenbart die Verwendung von wasserlöslichen oder in Wasser dispergierbaren Polyurethanen in kosmetischen und pharmazeutischen Zubereitungen. Die Polyurethane werden erhalten, indem in einem ersten Schritt ein Polyurethan-Intermediat aus einer Polyol-Komponente, einem Carbonsäureester und einem Polyisocyanat hergestellt wird. Dieses Intermediat wird anschließend durch Behandlung mit starken Basen verseift, wobei die Estergruppen in Säuregruppen überführt werden.

EP-A 043 974 offenbart antitumoral wirkende Mittel auf Basis wasserlöslicher Polyadditionsaddukte.

In der Kosmetik werden Haarbehandlungsmittel, die beispielsweise als Haarverfestiger oder Haarspray vorliegen, zum Festigen, Strukturverbessern und Formgeben der Haare verwendet. Die Haarbehandlungsmittel bestehen vorwiegend aus einer Lösung von filmbildenden Harzen oder synthetischen Polymeren. Bisher wurden in Haarbehandlungsmitteln hauptsächlich folgende Filmbildner verwendet: Schellack, Homo- und Copolymerisate des N-Vinylpyrrolidons, Copolymerisate von Vinylethern/Maleinsäurehalbestern, von (Meth)acrylsäure oder deren Estern und Amiden und Crotonsäure mit Vinylestern.

Die Haarbehandlungsmittel werden in Form von Lösungen, vorzugsweise als ethanolische Lösungen, durch Sprühen auf die Haare gebracht. Nach dem Verdampfen des Lösemittels werden die Haare an den gegenseitigen Berührungspunkten vom zurückbleibenden Polymer in der gewünschten Form gehalten. Die Polymeren sollen einerseits so hydrophil sein, daß sie aus dem Haar ausgewaschen werden können, andererseits sollen sie hydrophob sein, damit die mit den Polymeren behandelten Haare auch bei hoher Luftfeuchtigkeit ihre Form behalten und nicht miteinander verkleben.

Die bisher bekannten polymeren Filmbildner, wie Polyvinylpyrrolidone zeigen jedoch meistens als Nachteil eine zu hohe Wasseraufnahme bei erhöhter Luftfeuchtigkeit. Diese Eigenschaft führt u.a. zu einem unerwünschten Verkleben der Haare und zu einem Verlust der Festigkeit und damit einem Zusammenbruch der Haarfrisur. Wird andererseits die Widerstandsfähigkeit gegen hohe Luftfeuchtigkeit verbessert, z.B. bei Copolymerisaten aus N-Vinylpyrrolidon und Vinylacetat, so leidet darunter die Elastizität des Films und die Sprödigkeit dieser Filme kann nach der Haarbehandlung sogar zu einem unangenehmen Stauben und einem schuppigen Belag führen. Außerdem wird vor allem die Auswaschbarkeit bei der Reinigung der Haare sehr erschwert. Die obengenannten synthetischen Haarbehandlungsmittel sind aufgrund ihrer hydrolysebeständigen C-C-Kette biologisch nicht abbaubar. Schellack ist dagegen biologisch abbaubar, hat aber viele Nachteile. So sind seine Eigenschaften als Haarbehandlungsmittel im Vergleich zu den Homo- und Copolymerisaten des N-Vinylpyrrolidons schlechter, insbesondere bezüglich der Klebrigkeit, Wasserlöslichkeit und Steifigkeit. Da Schellack ein Naturprodukt ist, sind seine Eigenschaften starken Schwankungen unterlegen.

Der vorliegenden Erfindung liegt die Aufgabe zugrunde, Hilfsmittel für kosmetische und pharmazeutische Zubereitungen zur Verfügung zu stellen.

Die Aufgabe wird erfindungsgemäß gelöst mit der Verwendung von wasserlöslichen oder in Wasser disperglerbaren Polyurethanen aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat
mit einer Glastemperatur von mindestens 15°C und Säurezahlen von 12 bis 150 oder den Salzen dieser Polyurethane in kosmetischen Zubereitungen und als Bindemittel oder Überzugsmittel in pharmazeutischen Zubereitungen.

Für die erfindungsgemäße Verwendung kommen alle wasserlöslichen oder in Wasser dispergierbaren Polyurethane in Betracht, die die oben angegebenen Komponenten a) bis c) einpolymerisiert enthalten, eine Glastemperatur von mindestens 15°C und Säurezahlen von 12 bis 150 aufweisen sowie die Salze der Polyurethane. Als Verbindungen der Gruppe a) kommen alle die für die Herstellung von Polyurethanen einsetzbaren Verbindungen mit 2 oder mehreren aktiven Wasserstoffatomen pro Molekül in Betracht. Als Verbindungen der Gruppe a) eignen sich beispielsweise Diole, Diamine, Polyesterole, Polyetherole oder Mischungen der genannten Verbindungen, wobei bis zu 3 mol-% der genannten Verbindungen durch Triole oder Triamine ersetzt sein können. Geeignete Diole sind beispielsweise Ethylenglykol, Propylenglykol, Butylenglykol, Neopentylglykol, Polyetherole wie Polyethylenglykole mit Molekulargewichten bis zu 3000, Blockcopolymerisate aus Ethylenoxid und Propylenoxid mit Molekulargewichten nach dem Zahlenmittel von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Vorzugsweise verwendet man aus der Gruppe der Diole und Polyetherole Ethylenglykol, Neopentylglykol, Diethylenglykol, Triethylenglykol, Tetraethylenglykol, Pentaethylenglykol und Hexaethylenglykol.

Geeignete Diamine sind beispielsweise Ethylendiamin, Propylendiamin, 1,4-Diaminobutan und Hexamethylendiamin sowie α,ω-Diamine, die durch Aminierung von Polyalkylenoxiden, insbesondere Polyethylenoxiden mit Ammoniak herstellbar sind.

Als Verbindungen der Gruppe a) kommen außerdem sämtliche Polyesterole in Betracht, die üblicherweise zur Herstellung von Polyurethanen eingesetzt werden, z.B. Umsetzungsprodukte aus Phthalsäure und Diethylenglykol, Isophthalsäure und Butandiol-(1,4), Isophthalsäure/Adipinsäure und Hexandiol-(1,6) sowie aus Adipinsäure und Ethylenglykol.

Insbesondere eignen sich als Polyesterole Poly(α-Hydroxycarbonsäurediole) der Formel 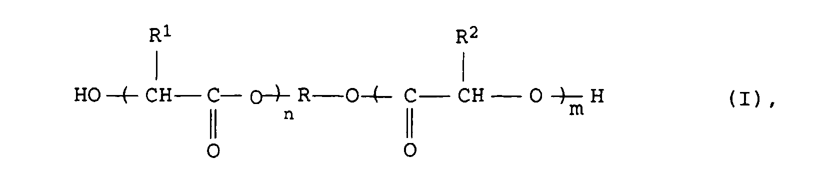 in der
- R¹, R²: H, C₁- bis C₅-Alkyl oder Aryl,
- R: Rest eines zweiwertigen Diols (Alkylenrest) mit 2 bis 8 C-Atomen
- n, m: 1 - 30 bedeuten.

Der Rest R in Formel I bedeutet vorzugsweise -CH₂-CH₂-, 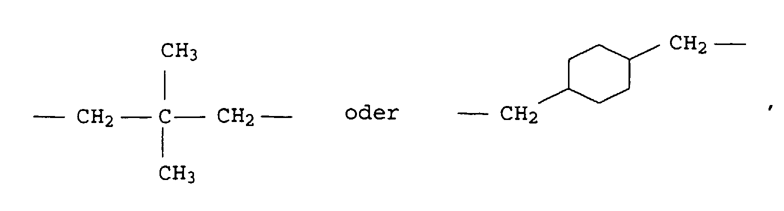 die Reste R¹ und R² stehen vorzugsweise für CH₃.

Geeignete α-Hydroxycarbonsäuren für die Herstellung der Poly-α-Hydroxycarbonsäurediole sind beispielsweise Milchsäure, α-Hydroxybuttersäure, Lactid und Glyoxylsäure. Vorzugsweise setzt man Milchsäure ein, von der sämtliche Isomeren geeignet sind: L,D,DL-Milchsäure.

Zur Herstellung der Polyurethane kann man auch Mischungen von Verbindungen der Gruppe a) einsetzen, z.B. Mischungen aus einem Diol und einem Polyesterol, oder einem Diol und Polyetherolen. In den Mischungen können bis zu 3 mol-% der genannten Verbindungen durch Triole oder Triamine ersetzt sein. Geeignete Triole sind beispielsweise Glycerin, Trimethylolethan oder Trimethylolpropan. Als Triamine eignen sich insbesondere Diethylentriamin oder Dipropylentriamin.

Als Verbindungen der Gruppe b) zur Herstellung der Polyurethane können alle hierfür üblichen Säure- oder Salzgruppen enthaltenden Diole eingesetzt werden. Insbesondere eignen sich Dimethylolpropansäure, Verbindungen der Formel 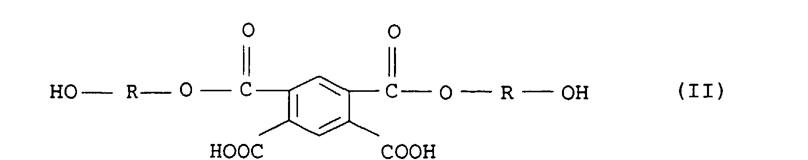 und/oder

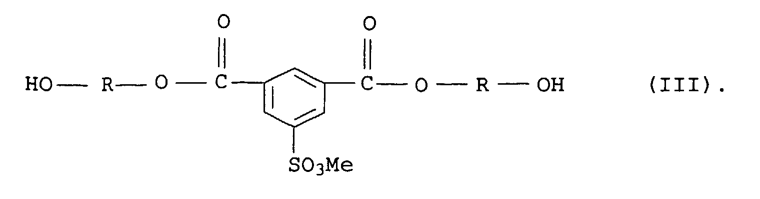

In den Formeln II und III steht R jeweils für eine C₂- bis C₁₈-Alkylengruppe und bedeutet vorzugsweise -CH₂-CH₂-, 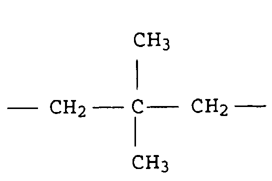 und/oder 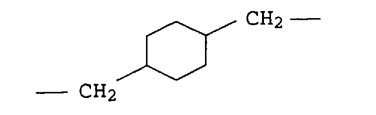 In Formel III steht Me für Na oder K.

Zur Herstellung der Polyurethane können die üblicherweise verwendeten Di- und Polyisocyanate verwendet werden. Besonders bevorzugt verwendet man als Verbindungen der Gruppe c) Hexamethylendiisocyanat, Isophorondiisocyanat und/oder Toluylendiisocyanat. Wie bei der Herstellung von Polyurethanen üblich, kann man Kettenverlängerer verwenden. Geeignete Kettenverlängerer sind beispielsweise Hexamethylendiamin, Piperazin, 1,2-Diaminocyclohexan, 1,3-Diaminocyclohexan, 1,4-Diaminocyclohexan, Neopentandiamin und 4,4'-Diaminodicyclohexylmethan.

Sämtliche oben beschriebenen Polyurethane werden erfindungsgemäß als Hilfsmittel in kosmetischen und pharmazeutischen Zubereitungen verwendet. Für die Anwendung im kosmetischen und pharmazeutischen Bereich werden diejenigen Polyurethane eingesetzt, die Säurezahlen von 12 bis 150, vorzugsweise 30 bis 90 sowie eine Glastemperatur von mindestens 15°C haben. Die Glastemperatur T_{g} kann bis zu 120°C betragen und liegt vorzugsweise in dem Bereich von 30 bis 100°C. Die Glastemperatur T_{g} wird nach ASTM D 3418 bestimmt.

Die Polyurethane sind nach Neutralisation (teilweise oder vollständig) wasserlöslich bzw. ohne Zuhilfenahme von Emulgatoren in Wasser dispergierbar. In aller Regel weisen die Salze der Polyurethane, die durch Neutralisation mit Basen daraus erhältlich sind, eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Besonders für den Einsatz in Haarbehandlungsmitteln haben sich zur Neutralisation der Säuregruppen enthaltenden Polyurethane 2-Amino-2-Methylpropanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z.B. Mischungen aus Natronlauge und Triisopropanolamin. Die Neutralisation kann je nach Anwendungszweck partiell z.B. zu 20 bis 40 % oder vollständig, d.h. zu 100 % erfolgen.

Sind die Verbindungen wasserdispergierbar, können sie in Form von wäßrigen Mikrodispersionen mit Teilchendurchmessern von üblicherweise 5 bis 100 nm, insbesondere 10 bis 80 nm, und Feststoffgehalten von üblicherweise 1 bis 40 Gew.-%, insbesondere 3 bis 30 Gew.-%, zur Anwendung gebracht werden. Diese Mikrodispersionen benötigen in der Regel keine Emulgatoren oder Tenside zu ihrer Stabilisierung.

Die von Milchsäurepolyolen abgeleiteten Polyurethane sind zumindest teilweise biologisch abbaubar. Sämtliche Säuregruppen enthaltenden Polyurethane sind am Klärschlamm zu mehr als 90 % eliminierbar (bestimmt nach Zahn-Wellens gemäß DIN 38 412, Teil 25).

Die oben beschriebenen Polyurethane werden außer in der Haarkosmetik auch für Cremes und im Pharmabereich als Tablettenüberzugsmittel und Tablettenbinder verwendet. Sofern die oben beschriebenen Polyurethane als Haarbehandlungsmittel verwendet werden, gelangen sie meistens in Form von wäßrigen oder ethanolischen Lösungen zur Anwendung. Der Feststoffgehalt dieser Lösungen beträgt 0,1 bis 30, vorzugsweise 1 bis 15 Gew.-% Polyurethan oder Salz eines Polyurethans.

### Beispiele

### Allgemeine Herstellungsvorschrift

In einem 4-Halskolben, der mit Rührer, Tropftrichter, Thermometer, Rückflußkühler und Vorrichtung für das Arbeiten unter Stickstoff ausgestattet ist, werden die in der Tabelle angegebenen Verbindungen a) und b) im Methylethylketon gelöst. Dazu wird das Reaktionsgemisch auf eine Temperatur von ca. 80°C unter Rühren erhitzt. Sobald sich alles gelöst hat, kühlt man das Reaktionsgemisch auf ca. 50°C ab und tropft unter Rühren das in der Tabelle unter c) jeweils angegebene Diisocyanat zu. Die Reaktionstemperatur steigt dabei an. Bei einer Innentemperatur von 90°C wird das Reaktionsgemisch dann solange gerührt, bis der Isocyanatgruppengehalt des Gemisches praktisch konstant bleibt. Danach kühlt man das Reaktionsgemisch auf eine Temperatur in dem Bereich von 10°C bis 30°C ab und tropft bei dieser Temperatur das in der Tabelle angegebene Diamin langsam zu. Man rührt das Reaktionsgemisch dann noch solange in diesem Temperaturbereich, bis der Isocyanatgruppengehalt auf 0 abgefallen ist. Sofern man keinen Kettenverlängerer zusetzt, werden die restlichen Isocyanatgruppen durch Zusatz von Aminen, z.B. 2-Amino-2-methyl-1-propanol inaktiviert. Man fügt dann Ethanol zu und entfernt den größten Teil des Methylethylketons und des Ethanols unter vermindertem Druck bei ca. 40°C. Das restliche Ethanol wird im Vakuumtrockenschrank bei 50°C entfernt. Man erhält nach dem Trocknen ein elastisches bis sehr hartes Produkt, das in Ethanol sowie in Wasser - vorzugsweise nach der Neutralisation mit einem Amin - löslich bzw. dispergierbar ist.

Anstelle der Zugabe von Ethanol zum Reaktionsgemisch kann man auch Wasser zusetzen und das Reaktionsprodukt neutralisieren, z.B. mit einem Amin. Das als Lösemittel verwendete Methylethylketon kann dann im Vakuum bei 40°C abdestilliert werden, so daß man direkt eine wäßrige Lösung bzw. Dispersion eines säuregruppenenthaltenden Polyurethans mit den in der Tabelle angegebenen Eigenschaften erhält. Die Abkürzungen in der Tabelle haben folgende Bedeutung:
- PEG300:: Polyethylenglykol M_{w} = 300 g/mol
- NPG:: Neopentylglykol
- DMPA:: Dimethylolpropansäure
- IPDI:: Isophorondiisocyanat
- P(IPS/ADS-VI):: Polyesterol mit M_{w} = 1000 g/mol aus Isophthalsäure, Adipinsäure und Hexandiol.
- P(ADS-DEG):: Polyesterol mit M_{w} = 500 g/mol aus Adipinsäure und Diethylenglykol
- P(PS-DEG) :: Polyesterol mit M_{w} = 450 g/mol aus Phthalsäure und Diethylenglykol
- P(MIS-EG) :: Polymilchsäure-ethylenglykol M_{w} = 500 g/mol.
- P(PMDA-NPG):: Kondensat aus Pyromellitsäuredianhydrid und Neopentylglykol vom Molekulargewicht M_{w} von ca. 430 mit der Struktur 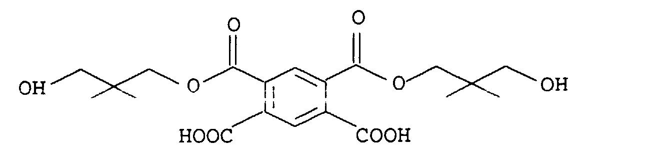
- P(SIPS-NPG):: Kondensat aus 5-Natriumsulfonato-isophthalsäure mit Neopentylglykol vom Molekulargewicht M_{w} ca. 440 und der Struktur 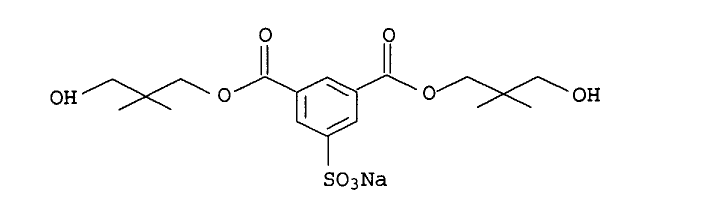
- NMP:: N-Methylpyrrolidon
- EtOH:: Ethanol
- l:: leicht löslich
- disp:: dispergierbar

Die biologische Abbaubarkeit der Polyurethane wurde nach Zahn-Wellens, DIN 38 412, Teil 25 bestimmt.

Die Polyurethane 1 bis 5 gehören zum Stand der Technik, während die Polyurethane 6 und 7 neue Stoffe gemäß Erfindung sind.

Um die Verwendung als Haarbehandlungsmittel zu demonstrieren, wurden folgende Haarbehandlungsmittel hergestellt:

| (a) Aerosol-Haarspray (rein ethanolisch) | |
|---|---|
| Polyurethan gemäß Beispiel 3 | 3% |
| 2-Amino-2-methyl-propanol | 0,26% |
| Ethanol abs. | 61,74% |
| Dimethylether | 35 % |

| (b) Aerosol-Haarspray (wäßrig-alkoholisch) | |
|---|---|
| Polyurethan gemäß Beispiel 3 | 3,00% |
| 2-Amino-2-methyl-propanol | 0,26% |
| Wasser dest. | 10,00% |
| Ethanol abs. | 51,74% |
| Dimethylether | 35,00% |

| (c) Handpumpenspray | |
|---|---|
| Polyurethan gemäß Beispiel 3 | 6,00% |
| 2-Amino-2-methyl-propanol | 0,52% |
| Wasser dest. | 93,48% |

| (d) Haarfestiger (rein wäßrig) | |
|---|---|
| Polyurethan gemäß Beispiel 5 | 4,00% |
| 2-Amino-2-methyl-propanol | 0,37% |
| Wasser dest. | 95,63% |

| (e) Haarfestiger (wäßrig-alkoholisch) | |
|---|---|
| Polyurethan gemäß Beispiel 5 | 4,00% |
| 2-Amino-2-methyl-propanol | 0,37% |
| Wasser dest. | 63,75% |
| Ethanol abs. | 31,88% |

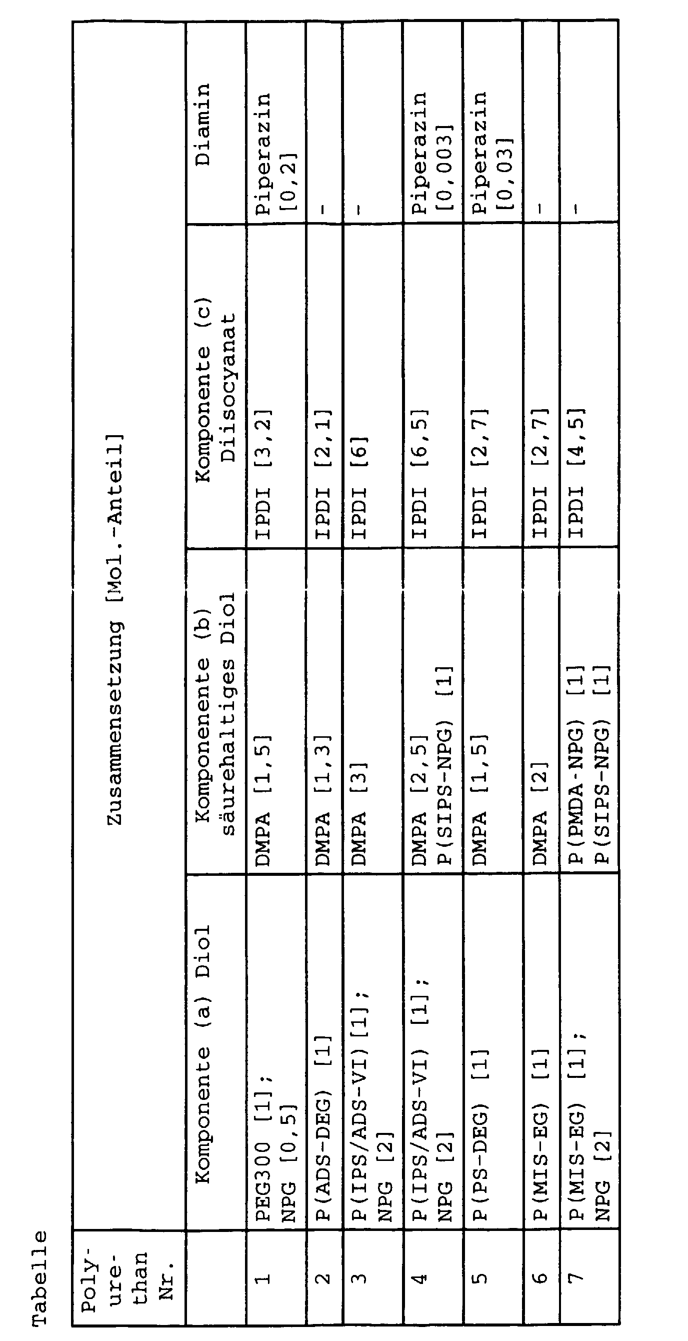

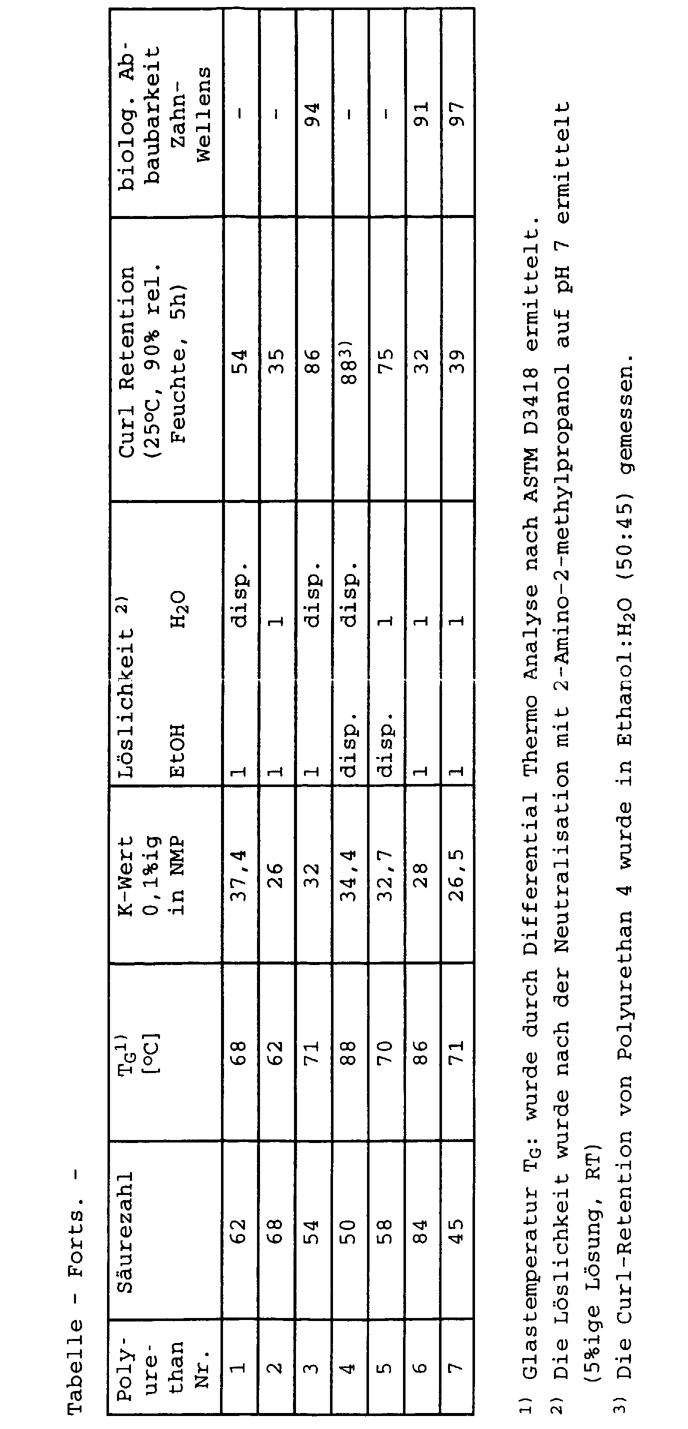

## Patentansprüche

1. Verwendung von wasserlöslichen oder in Wasser dispergierbaren Polyurethanen aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat
mit einer Glastemperatur von mindestens 15°C und Säurezahlen von 12 bis 150 oder den Salzen dieser Polyurethane in kosmetischen Zubereitungen und als Bindemittel oder Überzugsmittel in pharmazeutischen Zubereitungen.

2. Verwendung nach Anspruch 1, dadurch gekennzeichnet, daß man als Verbindungen der Gruppe (a) Diole, Diamine, Polyesterole, Polyetherole oder deren Mischungen mit einem Molekulargewicht (Zahlenmittel) von jeweils bis zu 3000 verwendet, wobei bis zu 3 mol-% der genannten Verbindungen durch Triole oder Triamine ersetzt sein können.

3. Verwendung nach Anspruch 1, dadurch gekennzeichnet, daß als Verbindungen der Gruppe (a) mindestens 20 mol-% Poly(α-hydroxycarbonsäurediole) der Formel 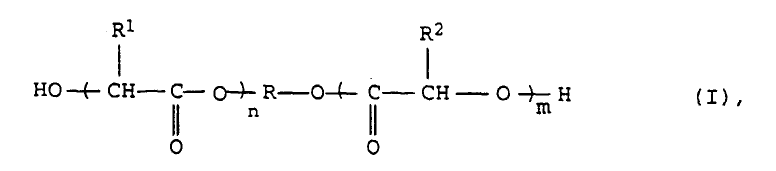 in der
R¹, R² H, C₁- bis C₅-Alkyl oder Aryl,
R Rest eines zweiwertigen Diols (Alkylenrest) mit 2 - 8 C-Atomen
n, m 1 - 30 bedeuten,
verwendet werden.

4. Verwendung nach Anspruch 1, dadurch gekennzeichnet, daß man als Verbindungen der Gruppe (b) Dimethylolpropansäure, Verbindungen der Formeln 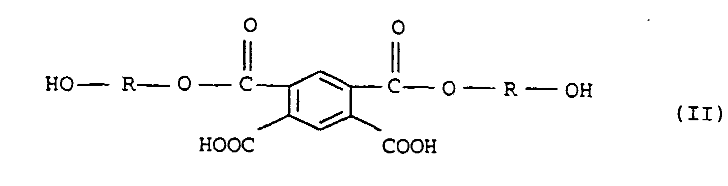 und/oder 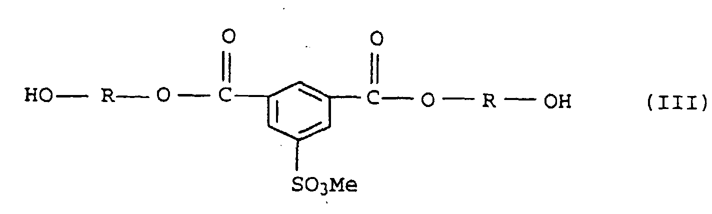 verwendet, in denen R jeweils für eine C₂- bis C₁₈-Alkylengruppe und Me für Na oder K steht.

5. Verwendung nach Anspruch 1, dadurch gekennzeichnet, daß man als Verbindungen der Gruppe (c) Hexamethylendiisocyanat, Isophorondiisocyanat und/oder Toluylendiisocyanat verwendet.

## Claims

1. The use of polyurethanes which are soluble or dispersible in water and are composed of
a) at least one compound which contains two or more active hydrogens per molecule,
b) at least one diol containing acid groups or salt groups and
c) at least one diisocyanate
with a glass transition temperature of at least 15°C and acid numbers of from 12 to 150 or the salts of these polyurethanes in cosmetic compositions and as binders or coating agents in pharmaceutical compositions.

2. The use as claimed in claim 1, wherein diols, diamines, polyesterols, polyetherols or mixtures thereof with a molecular weight (number average) of in each case up to 3000 are used as compounds in group (a), it being possible to replace up to 3 mol % of said compounds by triols or triamines.

3. The use as claimed in claim 1, wherein at least 20 mol % of poly(α-hydroxy carboxylic acid diols) of the formula 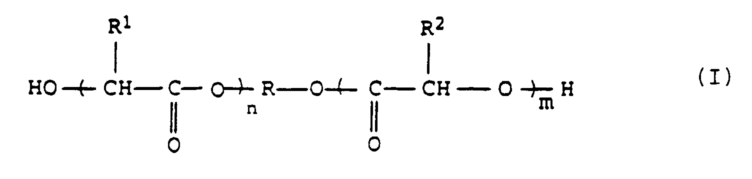 where
R¹ and R² are each H, C₁-C₅-alkyl or aryl,
R is a radical derived from a diol (alkylene radical) with 2 - 8 carbons,
n and m are each 1 - 30,
are used as compounds in group (a).

4. The use as claimed in claim 1, wherein dimethylolpropanoic acid, compounds of the formulae 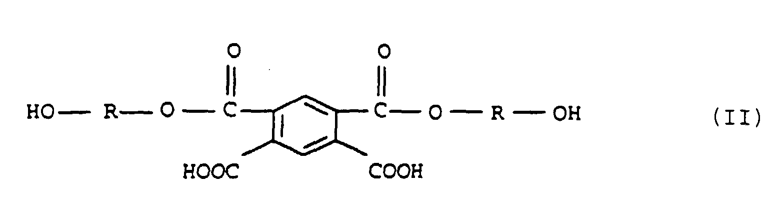 and/or 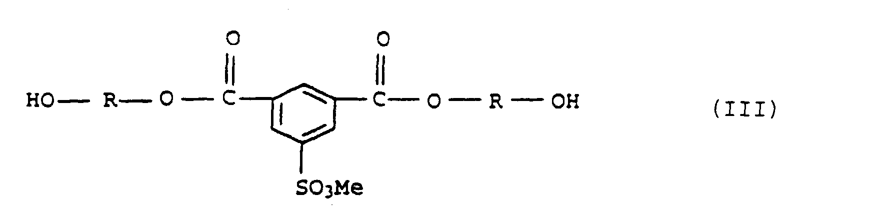 where R is in each case C₂-C₁₈-alkylene, and Me is Na or K, are used as compounds in group (b).

5. The use as claimed in claim 1, wherein hexamethylene diisocyanate, isophorone diisocyanate and/or toluylene diisocyanate are used as compounds in group (c).

## Revendications

1. Utilisation de polyuréthannes solubles dans l'eau ou dispersibles dans l'eau, qui se composent
a) d'au moins un composé qui contient deux ou plus de deux atomes d'hydrogène actifs par molécule,
b) d'au moins un diol contenant des radicaux acide ou sel et
c) d'au moins un diisocyanate,
avec une température de transition vitreuse d'au moins 15°C et des indices d'acidité de 12 à 150, ou des sels de ces polyuréthannes, dans des préparations cosmétiques et à titre de liants ou d'agents d'enrobage dans des préparations pharmaceutiques.

2. Utilisation suivant la revendication 1, caractérisée en ce que l'on utilise, à titre de composés du groupe (a), des diols, des diamines, des polyestérols, des polyéthérols, ou leurs mélanges, qui possèdent à chaque fois un poids moléculaire (moyenne en nombre) qui s'élève jusqu'à 3000, où jusqu'à 3% molaires des composés cités peuvent être remplacés par des triols ou des triamines.

3. Utilisation suivant la revendication 1, caractérisée en ce que l'on utilise, à titre de composés du groupe (a), au moins 20% molaires de poly(acides (α-hydroxycarboxyliquediols) de la formule 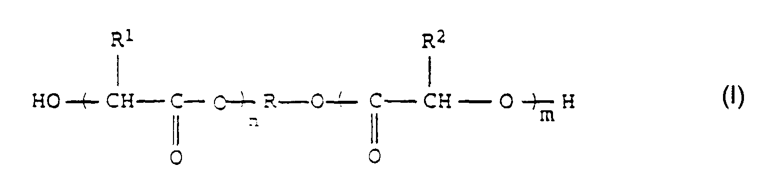 dans laquelle
R¹, R² représentent chacun un atome d'hydrogène, un radical alkyle en C₁ à C₅, ou un radical aryle,
R représente le reste d'un diol bivalent (reste alkylène) qui comporte de 2 à 8 atomes de carbone,
n, m sont des nombres dont la valeur varie de 1 à 30.

4. Utilisation suivant la revendication 1, caractérisé en ce que l'on utilise, à titre de composés du groupe (b), l'acide diméthylopropanoïque, des composés des formules 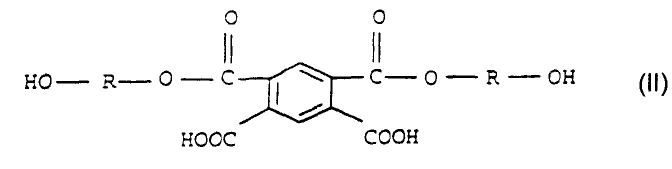 et/ou 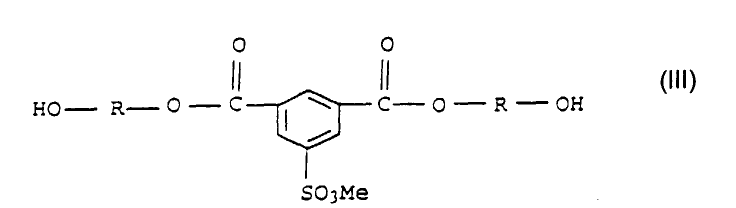 dans lesquelles R représente à chaque fois un radical alkylène en C₂ à C₁₈ et Me représente un atome de sodium ou de potassium.

5. Utilisation suivant la revendication 1, caractérisée en ce que l'on utilise, à titre de composés du groupe (c), le diisocyanate d'hexaméthylène, le diisocyanate d'isophorone et/ou le diisocyanate de toluylène.
